# FASCICULE DE BREVET EUROPEEN

(11) **EP 0 876 374 B1**
(45) Date de publication et mention de la délivrance du brevet: **24.07.2002**
(21) Numéro de dépôt: 96942404.3
(22) Date de dépôt: 13.12.1996
(51) Int. Cl.: C07D 493/04

(54) **PROCEDE DE PREPARATION DE LA 4'-DEMETHYLEPIPODOPHYLLOTOXINE A PARTIR DE LA PODOPHYLLOTOXINE**
HERSTELLUNGSVERFAHREN DES 4'-METHYLEPIPODOPHYLLOTOXIN AUS PODOPHYLLOTOXIN
METHOD FOR PREPARING 4'-DEMETHYLEPIPODOPHYLLOTOXIN FROM PODOPHYLLOTOXIN

(30) Priorité: 14.12.1995 FR 9514875
(43) Date de publication de la demande: 11.11.1998
(73) Titulaire: PIERRE FABRE MEDICAMENT, 92100 Boulogne-Billancourt (FR)
(72) Inventeur: IMBERT, Thierry, F-81290 Viviers-les-Montagnes (FR); GUMINSKI, Yves, F-81090 Lagarrigue (FR)
(74) Mandataire: Warcoin, Jacques
(86) Numéro de dépôt international: FR9602000
(87) Numéro de publication internationale: WO9721713

(56) Documents cités:
- WO-A-90/09788
- JOURNAL OF THE CHEMICAL SOCIETY, PERKIN TRANSACTIONS 1, 1977, LETCHWORTH GB, pages 2288-2289, XP002014444 N. FUJII ET AL.: cité dans la demande
- CHEMISTRY LETTERS, 1980, TOKYO JP, pages 875-878, XP002014445 H. IRIE ET AL.: cité dans la demande
- HETEROCYCLES, vol. 34, no. 5, 1992, pages 937-942, XP002014446 J. KUNITOMO ET AL.: cité dans la demande
- CHEMICAL ABSTRACTS, vol. 122, no. 19, 8 Mai 1995 Columbus, Ohio, US; abstract no. 240087q, XP002014451 & SYNTH. COMMUN., vol. 25, no. 3, 1995, pages 283-288, S. BERENYI ET AL.: cité dans la demande
- CHEMICAL ABSTRACTS, vol. 117, no. 23, 7 Décembre 1992 Columbus, Ohio, US; abstract no. 234312q, XP002014452 & SYNTH. COMMUN., vol. 22, no. 16, 1992, pages 2313-2327, J.-D. ANDRE ET AL.: cité dans la demande
- HELVETICA CHIMICA ACTA, vol. 52, no. 4, 1969, BASEL CH, pages 944-947, XP002014447 M. KUHN ET AL.: cité dans la demande
- JOURNAL OF MEDICINAL CHEMISTRY, vol. 29, 1986, WASHINGTON US, pages 1547-1550, XP002014448 L.S. THURSTON ET AL.: cité dans la demande
- ACTA CHEMICA SCANDINAVICA, vol. 47, 1993, COPENHAGEN DK, pages 1190-1200, XP002014449 H.F. HANSEN ET AL.: cité dans la demande
- CHEMISTRY LETTERS, 1987, TOKYO JP, pages 799-802, XP002014450 H. SAITO ET AL.: cité dans la demande

## Description

La présente invention concerne un nouveau procédé de déméthylation de la podophyllotoxine I pour accéder à la 4'-déméthylépipodophyllotoxine de formule II

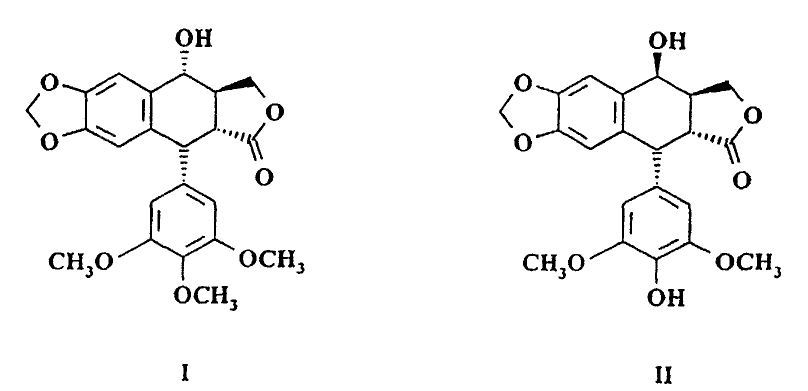

La 4'-déméthylépipodophyllotoxine est un intermédiaire clé de synthèse dans la préparation de nouvelles substances anticancéreuses, dont on peut citer par exemple l'Etoposide et le Téniposide.

La préparation de la 4'-déméthylépipodophyllotoxine II est connue et se fait de manière habituelle par déméthylation par HBr de la podophyllotoxine I, selon le procédé d'origine décrit par M. Kuhn, Helvetica Chimica Acta 1969, 52, 944, puis repris par plusieurs groupes tels que K.H. Lee, J. Med. Chem., 1986, 29, 1547, et Journal of Natural Products 1989, 52, 606, O. Buchardt, Acta Chemica Scandinavica 1993, 47, 1190, ou Y. Nishimura, Chemistry Letters 1987, 799. Ce procédé donne des rendements moyens selon les quantités mises en jeu et s'opère de façon relativement difficile, à cause de la manipulation de HBr gazeux très agressif. D'autres méthodes peuvent être envisagées pour effectuer cette déméthylation, en utilisant les réactifs usuels pour une telle transformation : on peut citer BBr₃ ou BCl₃ dans le CH₂Cl₂ de - 20°C à + 20°C (Tet. 1969, 24, 2289) ; AlCl₃ dans le CH₂Cl₂ à 0°C ou à température ordinaire (selon J. Chem. Soc. 1944, 330): le chlorhydrate de pyridinium à plus ou moins haute température : AlBr₃ en présence d'éthanethiol à température ordinaire.

Ces différentes méthodes conduisent à la dégradation ou à la non transformation de la podophyllotoxine.

Une nouvelle méthode de déméthylation a donc été mise au point.

Le procédé, objet de la présente invention, consiste à traiter la podophyllotoxine I par le couple de réactifs : acide fort - sulfure, en présence d'un acide organique ou minéral, ou bien en présence d'eau avec ou sans solvant organique miscible à l'eau, tel que le mélange acétone-eau, avantageusement, à une température comprise entre -10°C et + 40°C.

Selon des caractéristiques particulières de la présente invention, l'acide fort du couple de réactifs est avantageusement constitué par l'acide méthane sulfonique, alors que le sulfure du couple de réactifs est avantageusement choisi parmi les sulfures aliphatiques, la D,L-méthionine et l'acide méthylthioacétique.

Selon d'autres caractéristiques avantageuses du procédé de l'invention, la réaction est conduite en présence d'un acide organique de formule RCOOH où R = H, C₁-C₄ alkyle ou CX₃ avec X = Cl ou F. Il s'agira en particulier de l'acide formique ou de l'acide trifluoroacétique.

Dans le cas où la réaction est conduite en présence d'un acide minéral, on pourra avantageusement utiliser H₃ PO₄ ou encore un faible courant de HCl ou HBr barbotant dans le milieu réactionnel.

Conformément à la présente invention, le composé II directement issu de la réaction est isolé et purifié par recristallisation dans un solvant, tel que alcool isopropylique, acide acétique, eau, acétone, dioxane, éther isopropylique, toluène, éthanol et leur mélange.

La D,L-méthionine peut être également remplacée par un sulfure aliphatique, d'un faible nombre de carbone. Dans ce cas, la présence conjointe d'un acide minéral ou organique n'est plus forcément nécessaire.

Il est connu dans la littérature chimique que le couple acide méthane sulfonique, D,L-méthionine peut déméthyler les méthoxy aromatiques de certains composés pour conduire aux phénols correspondants:
voir J.C.S. Perkin Trans **I** 1977, 2288 ; J.C.S. Chem.Comm. 1976, 922; Chem. Lett. 1980, 875 ; J. Med. Chem. 1984, 27, 28 ; Heterocycles 1992, 34, 937 : Synth. Comm. 1992, 22 (16), 2313. Un des produits de la réaction est ainsi le méthylsulfonium dérivé de la méthionine, ce qui fait qu'un sulfure aliphatique ou arylique peut également être utilisé comme réactif de déméthylation, conduisant au sulfonium correspondant: voir à ce sujet: M. Julia Tet.Let. 1979, n°13, 1101.

Il apparait que cette méthode de déméthylation appliquée à la podophyllotoxine I ne conduit qu'à des produits de décomposition et par conséquent ne peut pas être utilisée.

Par contre si l'opération est conduite en présence d'un acide organique comme indiqué plus haut et en particulier l'acide formique, l'acide acétique ou l'acide trifluoroacétique, ou en présence d'un acide minéral en particulier l'acide phosphorique, ou en présence d'un solvant organique miscible à l'eau, comme le mélange acétone-eau, il est possible de déméthyler sélectivement le méthoxy en position 4' et d'obtenir le produit recherché avec un bon rendement.

L'acide minéral peut également être l'acide chlorhydrique ou bromhydrique gazeux, il est ainsi possible d'utiliser cette méthode, acide méthane sulfonique, D,L-méthionine sur podopyllotoxine I, où la fonction alcool en position 4 aura été préalablement transformée facilement dans des conditions douces, grâce à ce léger courant de gaz chlorydrique ou bromhydrique, en halogénure, Cl ou Br, ces opérations se faisant dans un procédé « one pot », avec le traitement par l'acide méthane sulfonique et la méthionine ; ces procédés sont décrits dans les exemples ci-après. Cette opération de traitement léger par Hbr gazeux n'est pas susceptible de déméthyler le méthoxy aromatique en 4', et n'utilise qu'une très faible quantité de gaz et pendant un temps très court, en regard du procédé antérieur.

L'avantage de cette méthode est sa simplicité de mise en oeuvre et la pureté du milieu réactionnel obtenu par rapport à la méthode antérieure, ne nécessitant pas la manipulation de gaz agressifs en grande quantité, et des installations particulières.

Les rendements de cette déméthylation en 4'-déméthylépipodophyllotoxine sont améliorés par rapport à l'art antérieur. Ce procédé peut s'appliquer à l'échelle industrielle sans difficultés particulières, par augmentation des quantités de substance mises en jeu, sans perte de rendement.

De plus, une amélioration supplémentaire du procédé réside dans le fait qu'il n'est pas nécessaire de purifier le composé obtenu, par chromatographie sur colonne, ce qui est une opération longue et coûteuse. Il est possible en fin de réaction de précipiter le produit formé dans l'eau et la glace, pour obtenir un produit cristallisé, qui est filtrable, et recristallisable dans des solvants comme par exemple l'isopropanol, l'éther isopropylique, l'acétone, l'acide acétique ou l'eau, et leur mélange. Il est possible aussi en fin de réaction d'extraire le produit par des solvants usuels de cristalliser et de recristalliser le résidu d'évaporation dans les solvants susmentionnés. Le produit de la réaction peut s'utiliser brut, même sans recristallisation, dans des étapes de synthèses ultérieures, comme par exemple la protection du phénol en position 4' par le chlorure de benzyloxy carbonyle.

Les exemples suivants illustrent les différents modes opératoires utilisés pour cette déméthylation:

| Exemple | Matière Première | Réactifs | Température | Temps | Rendement |
|---|---|---|---|---|---|
| 1 | 1 5 g | CH₃SO₃H (20 eq) D,L-méthionine (1,1 eq) | Température ordinaire | - | Dégradation |
| 2 | 1 12 g | CH₃SO₃H(45 eq) D,L-méthionine (5eq) CF₃CO₂H(6 eq) | 40° C | 0.75 h | 93 % |
| 2 bis | 1 100 g | CH₃SO₃H(45 eq) D,L-méthionine (5,5 eq) CF₃CO₂H(10 eq) | 0°C à 10-20° C | 1 h | 65 % |
| 3 | 1 7 g | CH₃SO₃H (37 eq) D,L-méthionine (8 eq) HCO₂H (4,5 eq) | 40° C | 25 mn | Rendement cristallisé : 48 % Rendement chromato : 48 % |
| 4 | 1 5 g | CH₃SO₃H(50 eq) D,L-méthionine (6 eq) H₃PO₄ | 0° C puis retour à température ordinaire | 6 h | 70 % |
| 5 | 1 1g | CH₃SO₃H(32 eq) D,L-méthionine (1,5 eq) Hcl gaz | - 10°C | 1 h | 52 % |
| 6 | 1 50 g | CH₃SO₃H (64 eq) D,L-méthionine (5,5 eq) Acétone-eau 5/1 | 40° C et retour à température ordinaire | 2 h | 80% |
| 7 | 1 5g | CH₃SO₃H (20 eq) D,L-méthionine (2 eq) Hbr gaz | - 10°C | 2 h | 54% |

### Préparation de la 4'-déméthylépipodophyllotoxine II

La podophyllotoxine utilisée est issue de source naturelle et présente une pureté de 95%.

### Exemple 1 Selon H. Yajima J. C.S. Perkin I 1977, 2288

1 g (2,4 mmoles) de podophyllotoxine I sont mis à réagir sous agitation avec 400 *mg* (2,64 mmoles) de D,L-méthionine et 3,1mL (48 mmoles) d'acide méthane sulfonique à température ordinaire. Le milieu se colore en rouge sombre immédiatement. La dégradation de la matière première est constatée par CCM.

### Exemple 2 Utilisation de l'acide trifluoroacétique

12 g (29 mmoles) de podophyllotoxine I sont dissous dans 20 ml d'acide trifluoroacétique et agités à température ambiante. On introduit 21,6 g (0,14 mole) de D,L-méthionine et 84 ml (1,29 mole) d'acide méthane sulfonique, la température s'élève à 40° C et l'agitation est maintenue pendant 45 mn. Le milieu réactionnel est alors jeté dans 500 ml d'une mélange en volume égal d'acétone et d'eau glacée. Neutraliser sous agitation jusqu'à pH 4 à 5 par addition de K₂ CO₃, puis extraire 2 fois par de l'acétate d'éthyle, sécher les phases organiques sur Na₂ SO₄, filtrer et évaporer le solvant sous pression réduite. Le résidu d'évaporation cristallise dans l'éther isopropylique 10,7 g Rdt 93 %.
La 4'-déméthylépipodophyllotoxine obtenue selon ce procédé peut être utilisée directement par la suite pour des réactions ultérieures telles que la protection du phénol en 4' par le chlorure de benzyloxycarbonyle.

### Exemple 2 bis Utilisation de l'acide trifluoroacétique

100 g (0.24 mole) de podophyllotoxine I sont dissous dans 186 ml d'acide trifluoracétique sous agitation. Après refroidissement à 0° C du milieu réactionnel, on introduit une solution de 198 g (1,32 moles) de D,L-méthionine dans 500 ml d'acide méthane sulfonique, en maintenant le milieu réactionnel entre 10 et 20° C. Rajouter 200 ml d'acide méthane sulfonique au mélange qui est gardé 1 h sous agitation à cette température. Le milieu réactionnel est versé sous agitation sur 4 l d'eau et de glace, le produit précipite. Il est extrait par l'acétate d'éthyle (3 x 1 l). Les phases organiques rassemblées sont lavées par une solution de NaHCO₃, puis séchées sur Na₂SO₄, filtrées et évaporées sous pression réduite pour obtenir 91,5 g (Rdt 94 %) de 4'-déméthylépipodophyllotoxine brute.

Bien que le produit puisse être utilisé directement dans les opérations suivantes de synthèse, le produit peut être purifié par chromatographie (SiO₂ CH₂Cl₂-acétone 95/5) pour fournir 65 % de 4'-déméthylépipodophyllotoxine pure.
Le produit brut de la réaction peut également être purifié par recristallisation dans l'acool isopropylique, l'acétone, l'acide acétique ou leur mélange avec de l'eau.

### Exemple 3 Utilisation de l'acide formique

7 g (17 mmoles) de podophyllotoxine I sont dissous dans 3,5 ml d'acide formique. une goutte d'acide méthane sulfonique est ajoutée et le milieu réactionnel est agité 15 mn à température ordinaire, on obtient une solution limpide jaune clair. On introduit alors sous agitation 10,5 g (68 mmoles) de D,L-méthionine et 49 ml (0,63 moles) d'acide méthane sulfonique, la température s'élève vers 40° C, pendant 10 mn. On ajoute à nouveau 10,5 g de D,L-méthionine au milieu réactionnel. Après 15 mn, le milieu est jeté dans 500 ml d'eau et de glace, on obtient un précipité qui est extrait 3 fois par de l'acétate d'éthyle, lavé avec une solution de NaHCO₃, séché sur Na₂SO₄ et évaporé pour obtenir 5,5 g du composé II (Rdt 81 %). Le produit obtenu est cristallisé dans le mélange éther isopropylique et CH₂Cl₂ pour obtenir 3,2 g de 4'-déméthylépipodophyllotoxine pure (Rdt 48 %). Une chromatographie effectuée sur le produit brut de réaction (SiO₂-élution CH₂Cl₂-90-acétone-10) fournit exactement le même résultat (3,2 g du dérivé II Rdt 48 %).

### Exemple 4 Utilisation de l'acide phosphorique

5 g (12 mmoles) de podophyllotoxine I sont rajoutés en une fois au mélange constitué d'acide phosphorique (2,5 ml) acide méthane sulfonique (40 ml, 0,6 moles) et D,L-méthionine (11 g, 72 mmoles) refroidi à 0° C sous agitation qui est maintenue pendant 6 heures avec retour à température ordinaire.

Le milieu réactionnel est alors jeté sous agitation sur 1 l d'eau et de glace pour obtenir un précipité blanc. Il est alors extrait par de l'acétate d'éthyle, trois fois puis lavé avec une solution de NaHCO₃. Les phases organiques sont rassemblées, séchées sur Na₂ SO₄, filtrées et évaporées sous pression réduite pour obtenir 4,2 g (Rdt 88 %) de 4'-déméthylépipodophyllotoxine II. La pureté du lot obtenu est de 79 % (analyse HPLC). Le résidu brut obtenu peut être recristallisé dans les solvants tels que le mélange acétone eau (5/2), toluène-éthanol (90/10) ou dioxane-éther isopropylique (50/50).

### Exemple 5 Utilisation d'un intermédiaire 4-chloro-4-désoxyépipodophyllotoxine

1 g de podophyllotoxine I est placé en solution dans un mélange de 15 ml de CH₂Cl₂ et 5 ml d'éther éthylique. Un léger courant de gaz chlorhydrique est introduit dans la solution pendant 15 mn à - 10° C, puis l'agitation est maintenue 1 h à cette température. Après évaporation, sans chauffer, du milieu réactionnel, on ajoute 0,54 g de D,L-méthionine puis 5 ml d'acide méthane sulfonique. Après dissolution et agitation 1 h avec retour à température ordinaire, le milieu réactionnel est jeté dans un mélange eau-acétone, puis additionné de BaCO₃ jusqu'à neutralité. Après extraction par de l'acétate d'éthyle, décantation, séchage sur Na₂SO₄, filtration et évaporation sous pression réduite, on obtient un résidu qui est cristallisé dans l'éther éthylique pour fournir 0,5 g (Rdt 52 %) de cristaux blancs de 4'-déméthylépipodophyllotoxine, présentant une tache homogène en CCM.

### Exemple 6 Utilisation du mélange acétone-eau

On ajoute une solution de 50 g (0,12 mole) de podophyllotoxine I. préparée dans 50 ml d'acétone, à un mélange constitué de 100 g (0,67 mole) de D,L-méthionine, de 500 ml (7,7 moles) d'acide méthane sulfonique et de 10 ml d'eau, sous agitation à température ordinaire. La température s'élève vers 40° C et l'agitation est maintenue 2 h avec retour à température ambiante. Le milieu est alors versé sur de la glace pour obtenir un précipité qui est extrait par de l'acétate d'éthyle (3 x 700 ml). Les phases organiques sont lavées par une solution de NaHCO₃, puis décantées, séchées sur Na₂SO₄, filtrées et évaporées sous pression réduite pour obtenir la 4'-déméthylépipodophyllotoxine se présentant sous forme d'un solide blanc, on obtient 45g (Rdt 94 %).

Ce résidu est repris dans l'éther isopropylique pour obtenir 38 g (Rdt 80 %) de 4'-déméthylépipodophyllotoxine. Le produit ainsi obtenu peut encore éventuellement être recristallisé dans un mélange acétone-eau, mais néanmoins, il peut être utilisé directement pour les stades de synthèses ultérieurs.

### Exemple 7 Utilisation d'un intermédiaire 4-bromo-4-désoxyépipodophyllotoxine

5 g de podophyllotoxine I sont placés en solution dans un mélange de 100 ml de CH₂Cl₂ et 35 ml de Et₂O sous agitation lente à - 10° C. Un léger courant de HBr gazeux est introduit dans la solution pendant 10 mn. A ce stade, le composé obtenu III (R = Br). est celui décrit par M. Kuhn, Helvetica chimica Acta, **52**, 944(1969). Le milieu réactionnel est alors évaporé sous pression réduite sans chauffer. On introduit ensuite 3,6 g (24 mmoles) de D,L-méthionine et 15,5 ml (0,24 mole) d'acide méthane sulfonique sous agitation et la réaction est maintenue ainsi 2 h à température ambiante. Le milieu réactionnel est introduit alors goutte à goutte à un mélange à volume égal d'acétone et d'eau saturée de NaHCO₃ pour neutraliser. Après 10 mn d'agitation, ajouter à l'eau et extraire par l'acétate d'éthyle. décanter, sécher les phases organiques sur Na₂SO₄, filtrer puis évaporer sous pression réduite pour obtenir 4,7 g (98 %) de 4'-déméthylépipodophyllotoxine brute. Une chromatographie sur silice (élution CH₂Cl₂-acétone 90-10) fournit 2,6 g de 4'-déméthylépipodophyllotoxine pure (Rdt 54 %).

## Revendications

1. Procédé de synthèse de la 4'-déméthylépipodophyllotoxine de formule II à partir de la podophyllotoxine de formule I, consistant à la traiter par un couple de réactifs, constitué par l'acide méthane sulfonique, d'une part, et un sulfure aliphatique, la D,L-méthionine ou l'acide méthylthioacétique d'autre part, en présence d'eau avec ou sans solvant organique miscible à l'eau.

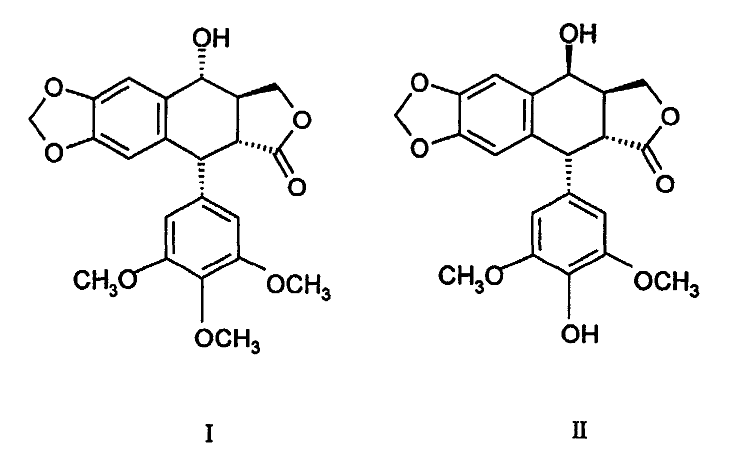

2. Procédé selon la revendication 1 ou 2, consistant à opérer à une température comprise entre -10°C et 40°C.

3. Procédé selon l'une des revendications 1 à 2, **caractérisé en ce que** le composé II directement issu de la réaction est isolé et purifié par recristallisation dans un solvant, tel que alcool isopropylique, acide acétique, eau, acétone, dioxane, éther isopropylique, toluène, éthanol et leur mélange.

## Patentansprüche

1. Verfahren zur Synthese von 4'-Desmethylepipodophyllotoxin der Formel II ausgehend von Podophyllotoxin der Formel 1, das darin besteht, dieses mit einem Paar Reagenzien, das einerseits aus Methansulfonsäure und andererseits aus einem aliphatischen Sulfid, D,L-Methionin oder Methylthioessigsäure zusammengesetzt ist, in Anwesenheit von Wasser mit oder ohne organisches Lösungsmittel, das mit Wasser mischbar ist, zu behandeln.

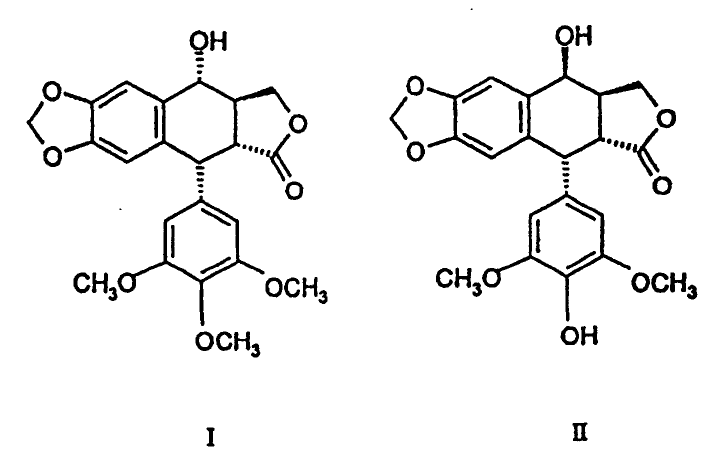

2. Verfahren nach Anspruch 1, das darin besteht, bei einer Temperatur von -10°C bis 40°C zu verfahren.

3. Verfahren nach einem der Ansprüche 1 bis 2, **dadurch gekennzeichnet, dass** die Verbindung II, die direkt aus der Reaktion hervorgeht, isoliert und durch Umkristallisation aus einem Lösungsmittel, wie Isopropylalkohol, Essigsäure, Wasser, Aceton, Dioxan, Isopropylether, Toluol, Ethanol und deren Mischung, gereinigt wird.

## Claims

1. Method for the synthesis of 4'-demethylepipodophyllotoxin of formula II from podophyllotoxin of formula I, which consists in treating it with a pair of reagents, consisting of methane sulphonic acid, on the one hand, and an aliphatic sulphide, D, L-methionine or methylthioacetic acid, on the other hand, in the presence of water, with or without water-miscible organic solvent

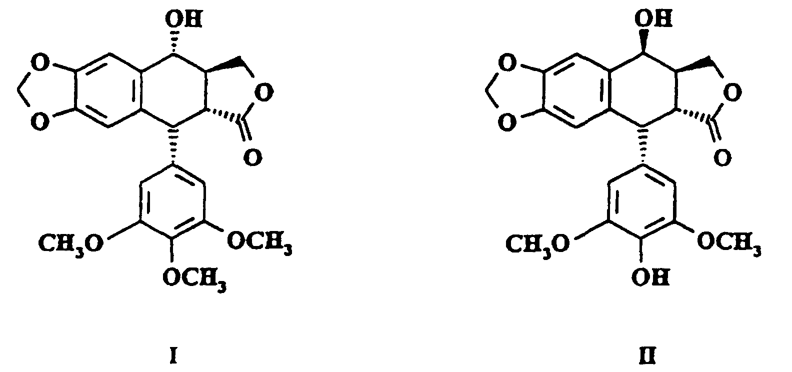

2. Method according to Claim 1, which consists in carrying out the synthesis at a temperature of between -10°C and 40°C.

3. Method according to either of Claims 1 and 2, **characterized in that** the compound II directly resulting from the reaction is isolated and purified by recrystallization from a solvent, such as isopropyl alcohol, acetic acid, water, acetone, dioxane, isopropyl ether, toluene, ethanol and their mixture.
